# EUROPEAN PATENT SPECIFICATION

(11) **EP 2 227 131 B1**
(45) Date of publication and mention of the grant of the patent: **27.03.2013**
(21) Application number: 07819676.3
(22) Date of filing: 02.11.2007
(51) Int. Cl.: A61B 3/107, G01B 11/25

(54) **METHOD OF EVALUATING A RECONSTRUCTED SURFACE AND CORNEAL TOPOGRAPHER**
VERFAHREN ZUR BEURTEILUNG EINER REKONSTRUIERTEN FLÄCHE UND HORNHAUT-TOPOGRAPH
PROCÉDÉ D'ÉVALUATION D'UNE SURFACE RECONSTRUITE ET TOPOGRAPHE CORNÉEN

(43) Date of publication of application: 15.09.2010
(73) Proprietor: Vereniging voor Christelijk Hoger Onderwijs, Wetenschappelijk Onderzoek en Patiëntenzorg, 1081 HV Amsterdam (NL)
(72) Inventor: DE PAZ SICAM, Victor Arni, 3082 ME Rotterdam (NL); SNELLENBURG, Joris-Joost, NL-1182 AR Amstelveen (NL); VAN DER HEIJDE, Gerrit Ludolph, NL-1399 GR Muiderberg (NL)
(74) Representative: De Weerdt, Robrecht
(86) International application number: PCT/EP2007/009666
(87) International publication number: WO 2009/056161

(56) References cited:
- WO-A-03/034910
- M. A.HALSTEAD, B.A. BARSKY, S.A. KLEIN, R.B. MANDELL: "Reconstructing Curved Surfaces from Specular Reflection Patterns Using Spline Surface Fitting of Normals" PROCEEDINGS OF THE 23TH ANNUAL CONFERENCE ON COMPUTER GRAPHICS AND INTERACTIVE TECHNIQUES, 1996, pages 335-342, XP002471240

## Description

### Field of the invention

### Corneal topography

### Background

Measurement of the corneal shape is becoming a common procedure in ophthalmic practice. This is done by a technique called keratoscopy. This technique allows the study of the corneal image and the interpreted image distortions as an indication of an abnormal cornea topography. In keratoscopy the following elements are used: a stimulator source to illuminate a target surface (e.g. the cornea of an eye) with a pattern of light and an image target arranged to receive the reflection of the light pattern. The information from the reflection is used to reconstruct the corneal shape. One of the most commonly used stimulator sources is the Placido ring pattern consisting of a disk with alternating black and white rings. In modern topographers, the reflected image of the target surface is captured by a camera and computer algorithms are applied to process this information to reconstruct the corneal shape. However, this procedure is not without problems. It can e.g. be noted that, when reconstructing the corneal surface, numerical algorithms used in commercially available Placido disk topographers neglect skew ray reflections. This leads to inaccuracy in reconstructing corneal surfaces that are not rotationally symmetric. In Placido disk topography, the corneal shape is reconstructed under the assumption that the reflection occurs in a meridian plane. However, this assumption is valid only if the corneal surface is rotation-symmetric. For non-rotation-symmetric surfaces, skew ray reflections can occur. This means that in Placido-based topography, there is ambiguity in the relationship between the stimulator source points and image points especially when the cornea is not a rotationally-symmetric surface. This ambiguity can e.g. be overcome by applying a stimulator source that enables to establish a one-to-one correspondence between a point on the stimulator source and a point on the image. For Placido-based topographers, this can be implemented by modifying the stimulator pattern to e.g. a checkerboard pattern. It can further be noted that when a colour-coded pattern is used instead of the Placido pattern, a similar correspondence between stimulator source points and image points can be obtained and skew ray ambiguity can be eliminated.

As explained above, topographer that are available today provide for a reconstruction of the target surface (e.g. the corneal surface), using numerical algorithms such as surface fitting using splines or Zernike polynomials. Until now, little attention has been given to developing techniques that allow an easy evaluation of the accuracy of the reconstructed surface. One known technique to evaluate the correctness of surface reconstruction algorithms is described by Halstead et al. in Optom. Vis. Sci. 1995, Vol. 72, pp. 821-827. The reconstructed corneal surface is evaluated by calculating the surface normals of the reconstructed surface and comparing them with the angle bisector between incident and reflected rays for each pair of source point and image point. For the correct surface these two vectors should be identical. If both vectors are not identical, a residual representing the difference between the two vectors can be defined. In the algorithm as described by Halstead, an angle residue (corresponding to the difference between the normal and the angle bisector) is calculated as residual. The residual can further be used to improve the accuracy of the reconstructed surface by e.g. a least-squares fitting. The document 'Reconstructing curved surfaces from specular reflection patterns using spline surface fitting of normals', M.A Halstead et al., Proceeding SIGGRAPH '96 p. 335-342, discloses images representing the reconstructed surface as provided by a calculation together with a mathematical target surface. A drawback of the proposed method is however that it does not provide an easy way to assess the relevance of the calculated difference between the actual (target) surface and the reconstructed surface.

### Object of the invention

Therefore, it is an object of the present invention to provide a method for evaluation of a reconstructed surface that enables to assess the relevance of the calculated difference between the actual (target) surface and the reconstructed surface in an easy manner. It is another object of the present invention to provide a corneal topographer that enables the evaluation of a reconstructed surface in an easy manner. It is a further object of the present invention to provide a calibration method for a corneal topographer.

Other objects and advantages of the present invention will become apparent from the description in which embodiments of the present invention are described.

### Summary of the invention

According to an aspect of the invention, there is provided a method of evaluating a correspondence between a target surface comprising a corneal surface and a reconstructed surface representing the target surface, according to claim 1.

By applying this method, the accuracy of the reconstructed surface can be assessed more easily because of the visualisation of the residual together with the image. By displaying the residual on the image target, one can also assess which parts of the reconstructed surface suffer from the largest error (or residual) and which parts have a small error. This may be important in case the accuracy requirement of the reconstructed surface is not uniform. As an example, reference can be made to the reconstruction of a corneal surface using a corneal topographer. In such an apparatus, a reconstruction of a corneal surface is determined (e.g. using a numerical algorithm), the reconstructed surface is further to be used in a subsequent surgical procedure to adjust a patients cornea e.g. by using laser technology. It will be clear to the skilled person that in order for this procedure to be successful, the accuracy of the reconstructed surface is important and should be verified. By visualising the reflected image of the target (e.g. the corneal surface), together with the residuals, the accuracy of the reconstructed surface can be assessed by visual inspection. This visual inspection may also be used to determine any further steps to be taken in e.g. modifying the reconstructed surface to reduce the discrepancy between the actual (target) surface and the reconstructed surface. Note that any method or device suitable can be applied for displaying the residual together with the reflected image. Both can e.g. be displayed on a screen that receives its input from a CCD camera, the camera serving as image target for receiving the reflected image from the target surface. Note that the image target can be any image (or picture) recording or receiving device such as a CCD camera or a video camera. The target surface in general represented the subject that is examined, in case the method is applied for examining a patients eye, the target surface can e.g. be the cornea of said eye.

According to another aspect of the present invention, there is provided a corneal topographer according to claim 7.

A corneal topographer according to the present invention differs from conventional topographer in that it enables a.o. a residual calculated from a comparison between an actual surface (e.g. a corneal surface) and a reconstructed surface to be displayed together with the reflected image from the target surface. Both can e.g. be displayed together on a screen of the topographer. As described above, such visual inspection being readily available provides an easy way to assess the accuracy of the reconstruction, it may also be a useful tool in deciding whether or not an adjustment of the reconstructed surface is required or not.

The method according to the invention, may be preceded by a calibration method, comprising the steps of
- identifying a reference image point on an image generated by reflecting a stimulator source on a reference surface towards an image target corresponding to a reference stimulator point,
- calculating the coordinates of the stimulator origin point of the reference image point by backward ray tracing from the image point towards the reference surface and from the reference surface towards the stimulator source,
- establishing the geometric relationship between the reference image point and the stimulator origin point.

In order for a corneal topographer to provide an accurate reconstructed surface of e.g. a corneal surface, a corneal topographer needs to be calibrated; the relative position between the different components of the topographer need to be know. As an example, surface reconstruction algorithms may depend on the position of the stimulator source relative to the image target being known. In case this relative position is not known or not sufficiently accurate, the calibration method according to the invention can be applied. As such, inaccuracies or errors occurring during an initial calibration by the manufacturer can be solved. The reference surface for use with the calibration method can e.g. be a substantially spherical surface having an knows geometry. The reference surface may also be a corneal surface of an eye of which the geometrical properties are known.

### Brief description of the figures

Figure 1 schematically depicts the basic principle of corneal topography;
Figure 2 schematically depicts a corneal topographer enabling a one-to-one correspondence between a stimulator point and an image point;
Figure 3 schematically depicts the principle of backward ray tracing as applied in an embodiment of the present invention;
Figure 4 schematically depicts the principle of pseudo-forward ray tracing as applied in an embodiment of the present invention.

### Figures

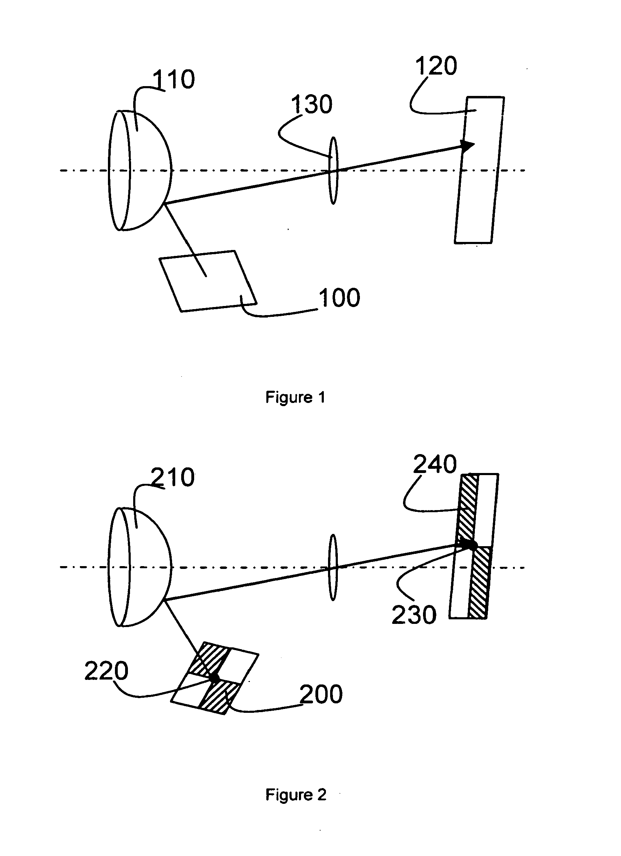

Figure 1 schematically depicts a part of a stimulator source 100 arranged to illuminate a target surface 110 (the target can e.g. be an eye). The ray of light emanating from the stimulator source is reflected on the target surface, the reflected ray of light is received by the image target 120 which can e.g. be a CCD camera. Between the target surface and the image target, a lens 130 can be present.
In general, when the stimulator source does not comprise distinct feature points, it is not possible to establish a one-to-one relationship between a point on the stimulator source and the corresponding projection on the image. Figure 2 schematically depicts an arrangement that enables this correspondence. Figure 2 schematically depicts a part of the stimulator source 200 arranged to illuminate a target surface 210. The part of the stimulator source 200 as shows comprises a pattern of rectangular shapes. The different parts or shapes of the stimulator source can e.g. emit a different colour. Alternatively, the pattern can be an alternating pattern of light and dark shapes. By doing so, a one-to-one correspondence between the stimulator source and the image can be obtained for the so-called crossings (in the stimulator source as depicted, the crossing corresponds to the point where the four rectangles meet) in the stimulator source. In figure 2, the crossing (also referred to as stimulator crossing SC) is indicated by reference number 220. As the pattern of the stimulator source is reflected on the target surface and projected on the image target, it will be clear to the skilled person that an image point 230 corresponding to the stimulator crossing can be found on the image 240. This image point is also referred to as the detected crossing DC.

Whether or not such a one-to-one correspondence between a number of stimulator source points and corresponding image points can be established depends on whether or not the stimulator source pattern comprises stimulator crossings that can result in identifiable crossings on the image targets. Topographers that enables such a one-to-one correspondence are e.g. topographers that project a checkerboard pattern, or a dartboard pattern or a colour-coded pattern.

Based on the geometric properties of the stimulator source, i.e. the position of the source relative to the surface target, and the image on the image target, a reconstruction of the target surface can be established. Different ways of achieving such a reconstructed surface exist, such as a curve fitting using Zernike polynomials or a fitting using spline functions.

Once such a reconstructed surface is established (e.g. as a continuous function describing the height of the cornea, or the height deviation from a spherical surface), this can be applied by a surgeon to adjust the shape of a patients cornea, e.g. using laser refractive surgery procedures. It will be clear that in such a procedure, the outcome may strongly depend on the correspondence between the actual target surface (i.e. the cornea of an eye) and the reconstructed surface.

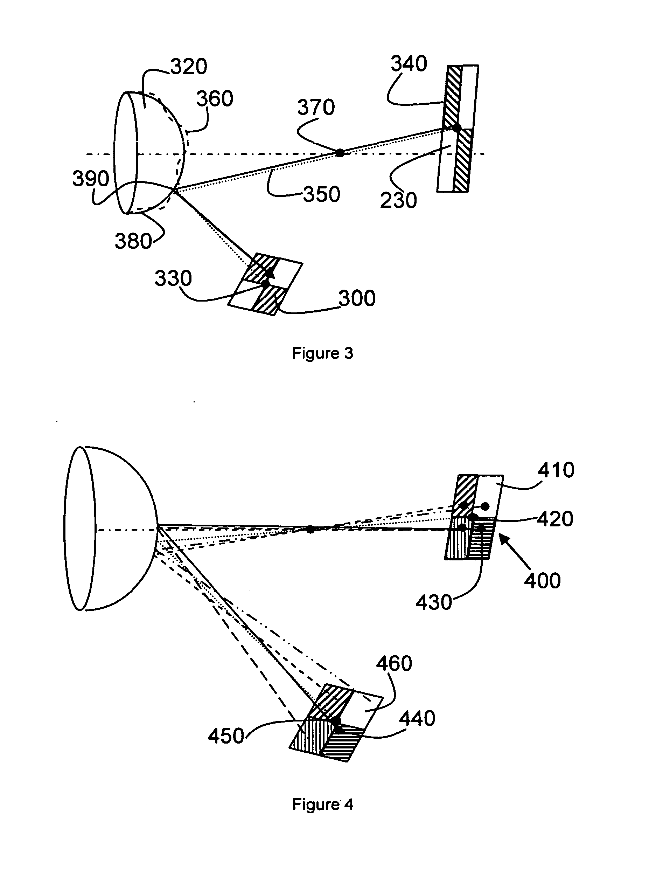

In case the topographer enables that such a one-to-one correspondence for a number of feature point (or crossings) is established, this can be applied to verify the accuracy of a reconstructed surface. One way to check the accuracy of the reconstructed surface is to use a back-ward trace algorithm to trace back the origin of an image point to the stimulator source using the reconstructed surface. This procedure is illustrated in figure 3. Figure 3 schematically a stimulator source 300 and an image target 310 of a corneal topographer. Also indicated in figure 3 are a stimulator crossing 330 and its corresponding detected crossing 340 (i.e. the location on the image target where a ray of light (indicated by the dotted line 350) emanating from the stimulator crossing 320 and reflected on the target surface would end up) on the image target 310. Note that the actual shape of the target surface 320 is indicated by the broken line 360. When the detected crossing 330 is established, it can be traced to the corresponding point (SC) on the stimulator via backward ray-tracing. In this procedure, a ray is traced back from a point on the image through the nodal point of the lens 370 to the reconstructed target surface (indicated by the solid line 380 of the target surface 320. The intersection point 390 on the reconstructed surface can, in general, be calculated because the surface is represented by an analytical function, e.g. as a combination of Zernike polynomials. The correctness of the reconstructed surface can be evaluated by comparing the bisector of the incident ray and reflecting ray with the normal vector on the intersection point. A difference between the two vectors (also referred to as the angle residue) is a measure for the error between the actual surface and the reconstructed surface. In general, any difference between the actual trajectory of a ray of light emanating from the stimulator source and its backward traced ray via the reconstructed surface, can be used as a measure indicating the accuracy of the reconstructed surface. Such a difference is referred to as a residual.

The residual and the reflected image are displayed together. This provides an easy way for e.g. a surgeon to assess the accuracy of the reconstructed surface and if required, take appropriate measures to improve the reconstructed surface.

According to an embodiment of the present invention the residual comprises an angle residual calculated by backward tracing the reference image point towards the reconstructed surface and from the reconstructed surface towards the stimulator source. The angle residual can e.g. be calculated as indicated above.

According to another embodiment of the present invention, an alternative method to verify the correctness of the surface reconstruction procedure is employed. The procedure is referred to as a pseudo-forward ray tracing (PFRT) routine. The procedure as described in figure 3 is referred to as a back-ward trace algorithm because an image point (the detected crossing) is traced back and compared with its point of origin. A forward ray tracing algorithm would trace the stimulator crossing to the image, however, as there are infinitely many rays emanating from the stimulator crossing, forward ray-tracing from this point to the corneal surface would be impossible.
To overcome this, the alternative method according to an embodiment of the present invention applies multiple backward ray-tracing procedures. According to an embodiment of the method, a region around each DC on the image target is considered (in case the image target is a CCD camera plane, the selected region can e.g. be a square region of a predefined number of pixels, e.g. 11 x 11 pixels). In such an arrangement, each pixel in this region is traced back to the stimulator source using a backward trace algorithm (as e.g. shown in figure 3). The pixel with the closest projection to the SC is considered the pixel location of the so-called residual crossing RC. The distance between the residual crossing RC and the detected crossing DC on the image plane can be considered a measure of the accuracy of the reconstructed surface (i.e., this distance can be considered a residual). A visualization of this procedure is shown in Figure 4. In this figure, 5 pixel points (400) on the image plane 410 are shown: the DC (420) and 4 pixel points in the neighbourhood of the DC. When traced back, pixel 430 is found to have the closest projection (440) to the SC 450 on the stimulator source 460. Thus, pixel 430 is considered as the residual crossing RC. Note that, for clarity, the distance between the pixels, nor the distance to the target surface or stimulator source is not up to scale with the target surface. Note also that the reconstructed surface that is used for backward tracing the different pixels is not separately shown in figure 4.

As will be apparent, the proposed method equally provides the possibility of displaying residual information about the accuracy of the reconstructed surface together with the reflected image. Such an on-screen evaluation of the residual (or residual information) together with the image enables an easy assessment of reconstructed surface. As the residual information can be shown together with the reflected image of e.g. the patients eye, one can assess whether or not the reconstructed image is sufficiently accurate, based on the size of the error (measured in e.g. a number of pixels) and the location of the error.

Regarding the latter aspect, it will be clear to the skilled person that a certain error of the reconstructed surface can be acceptable on certain locations of the cornea whereas the same error is unacceptable on other locations. The proposed evaluation method therefore provides the possibility of easily assessing in which areas the reconstructed surface needs further improvement.

A further advantage of the proposed method is that the amplitude of the error (e.g. the distance in pixels between the residual crossing RC and the detected crossing DC is found to be proportional to the corneal height accuracy. Depending on a.o. the pixel size, one can establish the relationship between the error at a certain location on the image plane (expressed in pixels) and the height accuracy (i.e. the distance between the actual (corneal) surface and the recomputed surface in a direction perpendicular to the surface) at that location. As an example, an error of e.g. one pixel may correspond to an height error of 1 micron. Based on this relationship, the proposed alternative method provides a further possibility to easily assess whether a certain error is acceptable or not.

According to yet a further embodiment of the present invention, the residual information obtained by the PFRT algorithm (as indicated in figure 4) or the backward trace algorithm (as explained in figure 3) is used to further improve the accuracy of the reconstructed surface. As such, the method of evaluating the correspondence between a target surface and a reconstructed surface representing the target surface may comprise the step of modifying the reconstructed surface based on calculated residual.

As an example, it is assumed that the target surface is reconstructed using the well-known Zernike polynomials. A combination of these polynomials (i.e. a summation of the different polynomials weighted by multiplying each polynomial with a corresponding Zernike coefficient) can be used to represent the target surface (e.g. the height of a cornea) as an analytical function. The required coefficients for the weighing of the Zernike polynomials can e.g. be obtained from a least-squares fitting routine. Because initially the surface is unknown, the initial Zernike coefficients can be set equal to zero, describing a flat surface. For each detected crossing on the image, an angle residue can be calculated as the difference between a normal vector (at the intersection point) and an angle bisector between incident and reflected ray, see figure 3. The calculated angle residues (or residuals) can then be used in a least-squares fitting procedure to determine a better model for the surface.

As will be clear to the skilled person, the residual of the detected crossings as obtained from the PRFT algorithm can equally be applied in a least-squares fitting routine for obtaining an improved surface. Compared to the use of the angle residues in an optimisation (or further improvement) routine, the use of the residuals of the PRFT algorithm provides the following advantages:
- as there is a known relationship between the residual (e.g. expressed in a number of pixels or pixel size) and the height accuracy (as explained above), the calculated residuals can be used to determine whether the optimisation process can be stopped or whether a further iteration is required. In case the residual values are below a certain value, one can easily determine the corresponding height accuracy. Alternatively, one can require a certain height accuracy (e.g. an corneal height error less than 1 micron) and determine the corresponding allowable residual value. This value can be used in the surface optimisation procedure as a criterion to stop or continue the procedure.
- As the PRFT algorithm also provides information as to where the errors occurs on the corneal surface, a weight function can be applied to the residuals such that an improvement in a next iteration focuses on the areas where an error is least acceptable.
It can further be noted that such a weight function may also be applied to suppress the contribution of large residuals (mainly outliers) in the fitting procedure.

The PFRT method as described above has been applied to measurements of five different surfaces:
(1) a PMMA (polymethyl methacrylate) spherical surface with 6.99 mm radius of curvature.
(2) a PMMA spherical surface with 9.00 mm radius of curvature.
(3) a PMMA toric surface with maximum axial radius of curvature of 8.02 mm and minimum axial radius of curvature of 7.05 mm.
(4) a human cornea, from the left eye of a 38-year-old man, with no known abnormality, and (5) a human cornea, from the left eye of a 61-year-old man, with subepithelial infiltrate.
As described, the PFRT method can produce residual information in pixel units of the reconstructed surface on the image (e.g. a CCD image) itself. To produce an accurate description of the corneal surface, two things must happen. First, the location of the image crossings (detected crossings DC) must be determined accurately. Second, the numerical reconstruction of the corneal surface must be consistent with the DCs. The output of the PFRT routine is an indicator whether the second procedure was implemented well. It will be clear to the skilled person that the accuracy of the first procedure (obtaining the position of the detected crossings DC) is important to obtain a reliable output of the PFRT procedure or any other evaluation method. In this respect, reference can be made to Spoelder HJW, Vos FM, Petriu EM, Groen FCA. Some aspects of pseudo random binary array-based surface characterization. IEEE Trans. Instrum. Meas. 2000;49:1331-6 showing that a subpixel accuracy in detecting the location of image crossings DC can be obtained. In case a 1 pixel would correspond to an height accuracy of 1 micron, the PFRT procedure can result in a submicrometer corneal height accuracy when the optimisation is continued until the calculated residuals are less than 1 pixel. In this respect, it is worth mentioning that the accuracy that can be obtained also depends on the complexity of the actual surface combined with the degrees of freedom of the surface fitting function. It has been shown that the overall residual (or mean residual of the detected crossings) increases with complexity of the measured surface. The residuals were found to be the smallest for the artificial surfaces; the spherical surfaces (1) and (2) resulted in a mean residual of 0.70 pixel, the toric surface (3) resulted in a mean residual of 0.81 pixel. The regular cornea (4) was found to have a slightly higher residual compared with the artificial surfaces (a mean residual of 1.16 pixel). This effect is found to be caused by the effect of higher order shape features. However, because these shape features are not as dominant when compared with the spherical and toric shape features, the effect on the residual was found to be relatively small. Whereas, for the irregular cornea (5), the effect of the higher-order shape features is larger, thus producing an increase in the mean value of the residual (a mean residual of 2.94 pixel was found when the surface was modelled using Zernike polynomials until radial order 6). The accuracy of the surface reconstruction was found to improve when the Zernike radial order used to model the corneal surface is increased. The addition of more Zernike components enables better fitting of the local surface features. For the artificial surfaces, a lower radial order for the Zernike expansion (order 6) is sufficient to reconstruct the surface with subpixel accuracy. For the regular cornea, subpixel accuracy was observed only for Zernike radial order of 10 or higher. For the irregular cornea (5), order 20 was found still not sufficient to produce subpixel accuracy for the surface reconstruction. Nevertheless, at this order the accuracy was found to approach pixel resolution, which is reasonable enough for clinical practice. The above also indicates that to some extent the use of Zernike polynomials will produce accurate corneal surface reconstruction as long as a sufficient radial order is used.

It should be noted that the PRFT routine as described does not depend on the way the reconstruction of the surface is done. It will be clear to the skilled person that any surface fitting procedure can be applied to provide a reconstructed surface. This reconstructed surface can then be evaluated using the PFRT routine as described and/or can be further optimised using the outcome of the PFRT routine (i.e. the residual information).

According to an embodiment of the present invention, there is provided in a corneal topographer that enables an evaluation of a reconstructed surface as described above. In order to do so, the corneal topographer comprises a computational unit arranged to
- construct a reconstructed surface representing the target surface by processing information of the stimulator source of the topographer and the image received on the image target of the topographer,
- identify a reference image point on the image target corresponding to a reference stimulator point on the stimulator source,
- calculate for the reference image point, using the reconstructed surface, a residual representing the correspondence between the target surface and the reconstructed surface,
- display the residual together with the reflected image.

As will be clear from the above, various way of determining the reconstructed surface or the residual can be applied in such a computational unit. It can further be noted that, in order to identify a reference image point on the image target of the topographer corresponding to a reference stimulator point on the stimulator source of the topographer, various types of stimulator sources can be applied. Examples of such stimulator sources are sources who provide a checkerboard (or dartboard) pattern of light or a colour coded pattern of light. Such a pattern can be obtained by applying different hue-values for the different area's of the pattern, or a different brightness or intensity. A colour coded pattern can be compared to a checkerboard that used areas of different colours as alternative to or in addition to areas that are black or white (i.e. dark and light). The various areas of different colours can be arranged in such manner that a reference image point on the image target of the topographer corresponding to a reference stimulator point on the stimulator source of the topographer can be found more easily.

The method according to the present invention, may be preceded by a calibration method. As explained above, an accurate construction of the reconstructed surface relies on accurate knowledge of the relative position of stimulator source and image target. In order to obtain this knowledge, the topographer can be used with a reference surface as target surface, rather than an unknown surface. Assuming the reference surfaces geometry is known, a corresponding reconstructed surface is also known. Applying any of the tracing routines as explained above on such a surface, should result in a residual substantially equal to zero. If a non-zero residual is found, this means that the initial assumption regarding the geometric relationship between the stimulator source and the image target was incorrect. As the reconstructed surface corresponds provides an accurate representation of the reference surface, backward ray tracing the reference image point towards the stimulator source (via the reconstructed surface) enables the actual co-ordinates of the reference stimulator point (relative to the image target) to be determined. As such, the actual position of the reference stimulator point relative to the corresponding reference image point can be established. It will be clear to the skilled person that in order to obtain the relative position between the stimulator source and the image target in all 6 degrees of freedom, the calibration can be performed for a plurality of reference stimulator points.

Although the examples that are described relate in particular to corneal topography, it can be stated that the methods as described (either the calibration method or the method for evaluating a reconstructed surface) may also be applied in other field of technology where accurate knowledge of the shape of a target surface is required. An example of such a field being semiconductor technology wherein an accurate knowledge of the surface characteristics of a substrate (such as a wafer) is required. Another field where the described methods may be applied is biometrical identification using e.g. an iris scan.

It can further be stated that the method of evaluating the correspondence between a target surface and a reconstructed surface representing the target surface may advantageously be combined with the calibration method as described. Since the calibration method enables an accurate determination of the geometrical relationship between the various components of the topographer, it may be advantageous to apply this calibration prior the reconstructed surface evaluation method as the geometric relationship between the stimulator source and the image target is used in determining the reconstructed surface.

## Claims

1. Method of evaluating a correspondence between a target surface comprising a corneal surface and a reconstructed surface representing the target surface, the reconstructed surface being constructed by processing information obtained by illuminating the target surface with a pattern of light of a stimulator source, and capturing a reflected image of the pattern of light on an image target, the method comprising the steps of
- determining a reference image point on the image target corresponding to a reference stimulator point on the stimulator source,
- calculating for the reference image point, using the reconstructed surface, a residual representing the correspondence between the target surface and the reconstructed surface,
- displaying the residual together with the reflected image of the target surface comprising the corneal surface,
- evaluating the correspondence between the target surface and the reconstructed surface from the displayed residual.

2. Method according to claim 1 wherein the residual comprises an angle residual calculated by backward tracing the reference image point towards the reconstructed surface and from the reconstructed surface towards the stimulator source.

3. Method according to claim 1 wherein the step of calculating the residual comprises the steps of
- calculating a plurality of stimulator points corresponding to a plurality of image points near the reference image point by backward tracing the image points towards the reconstructed surface and from the reconstructed surface towards the stimulator source ,
- selecting the image point who's corresponding stimulator point is closest to the reference stimulator point as the reconstructed reference image point origin,
- establish a residual from the difference between the reference image point and the reconstructed reference image point origin.

4. Method according to claim 2 or 3 wherein the construction of the reconstructed surface comprises a surface fitting using Zernike Polynomials.

5. Method according to claim 2 or 3 wherein the construction of the reconstructed surface comprises a surface fitting using spline functions.

6. Method according to any preceding claim further comprising the step of modifying the reconstructed surface based on calculated residual.

7. Corneal topographer comprising
- a stimulator source (300) arranged to, in use, illuminate a target surface (320) comprising a corneal surface with a pattern of light,
- an image target (310) arranged to receive the reflected image of the target surface, the corneal topographer further comprising a computational unit arranged to;
- construct a reconstructed surface (380) representing the target surface by processing information of the stimulator source and the image received on the image target,
- identify a reference image point on the image target corresponding to a reference stimulator point on the stimulator source,
- calculate for the reference image point, using the reconstructed surface, a residual representing the correspondence between the target surface and the reconstructed surface,
**characterized in that** the computational unit is arranged to
- display the residual together with the reflected image of the target surface comprising a corneal surface.

8. Corneal topographer according to claim 7 wherein the computational unit is arranged to calculate the residual by
- selecting a region on the image target surrounding the reference image point,
- calculating the corresponding stimulator origin point for a plurality of image points inside the region using back-ward tracing from the image point towards the reconstructed surface and from the reconstructed surface towards the stimulator source ,
- select the image point who's corresponding stimulator origin point is closest to the reference stimulator point as the reconstructed reference image point origin;
- establish the residual from the difference between the reference image point the reference image point and the reconstructed reference image point origin,

9. Corneal topographer according to claim 7 or 8 wherein the stimulator source is arranged to illuminate the target surface with a checkerboard pattern.

10. Corneal topographer according to claim 7 or 8 wherein the stimulator source is arranged to illuminate the target surface with a colour-coded pattern.

11. Corneal topographer according to any of claims 7 to 10 wherein the computational unit is further arranged to perform the step of modifying the reconstructed surface based on the calculated residual.

12. Corneal topographer according to claim 11 wherein the modification step includes a least squares fitting based on the calculated residual.

13. Method according to any of claims 1 to 6 wherein the method is preceded by a calibration method comprising the steps of
identifying a reference image point on an image generated by reflecting a stimulator source pattern on a reference surface towards an image target corresponding to a reference stimulator point,
calculating the coordinates of the reference stimulator point of the reference image point by backward ray tracing from the image point towards the reference surface and from the reference surface towards the stimulator source,
establish the geometric relationship between the reference image point and the reference stimulator point.

14. Corneal topographer according any of the claims 7 to 12 wherein the computational unit is further arranged to, in use, perform a calibration method comprising the steps of
identifying a reference image point on an image generated by reflecting a stimulator source pattern on a reference surface towards an image target corresponding to a reference stimulator point,
calculating the coordinates of the reference stimulator point of the reference image point by backward ray tracing from the image point towards the reference surface and from the reference surface towards the stimulator source,
establish the geometric relationship between the reference image point and the reference stimulator point.

## Patentansprüche

1. Verfahren zum Auswerten einer Übereinstimmung zwischen einer Zieloberfläche, die eine Hornhautoberfläche umfasst, und einer rekonstruierten Oberfläche, welche die Zieloberfläche repräsentiert, wobei die rekonstruierte Oberfläche durch Prozessieren von Information, die durch Beleuchten der Zieloberfläche mit einem Lichtmuster einer Stimulatorquelle und Erfassen eines reflektierten Bildes des Lichtmusters auf einer Bildzielvorrichtung erhalten wird, konstruiert wird, wobei das Verfahren die Schritte umfasst:
- Bestimmen eines Referenzbildpunktes auf der Bildzielvorrichtung, der einem Referenzstimulatorpunkt auf der Stimulatorquelle entspricht,
- Berechnen eines Residuums für den Referenzbildpunkt, unter Verwendung der rekonstruierten Oberfläche, welches die Übereinstimmung zwischen der Zieloberfläche und der rekonstruierten Oberfläche repräsentiert,
- Darstellen des Residuums zusammen mit dem reflektierten Bild auf der Zieloberfläche, die die Hornhautoberfläche umfasst,
- Auswerten der Übereinstimmung zwischen der Zieloberfläche und der rekonstruierten Oberfläche aus dem dargestellten Residuum.

2. Verfahren gemäß Anspruch 1, wobei das Residuum ein Winkelresiduum umfasst, welches durch Zurückverfolgen des Referenzbildpunktes hin zu der rekonstruierten Oberfläche und von der rekonstruierten Oberfläche hin zu der Stimulatorquelle berechnet wird.

3. Verfahren gemäß Anspruch 1, wobei der Schritt des Berechnens des Residuums die Schritte umfasst:
- Berechnen einer Vielzahl von Stimulatorpunkten, die einer Vielzahl von Bildpunkten nahe dem Referenzbildpunkt entsprechen, durch Zurückverfolgen der Bildpunkte hin zu der rekonstruierten Oberfläche und von der rekonstruierten Oberfläche hin zu der Stimulatorquelle,
- Selektieren des Bildpunktes, dessen zugehöriger Stimulatorpunkt am nächsten zu dem Referenzstimulatorpunkt ist, als der rekonstruierte Referenzbildpunktursprung,
- Bestimmen eines Residuums aus der Differenz zwischen dem Referenzbildpunkt und dem rekonstruierten Referenzbildpunktursprung.

4. Verfahren gemäß Anspruch 2 oder 3, wobei das Konstruieren der rekonstruierten Oberfläche eine Oberflächenanpassung unter Verwendung von Zernike-Polynomen umfasst.

5. Verfahren gemäß Anspruch 2 oder 3, wobei das Konstruieren der rekonstruierten Oberfläche eine Oberflächenanpassung unter Verwendung von Spline-Funktionen umfasst.

6. Verfahren gemäß einem voranstehenden Anspruch, weiterhin den Schritt umfassend: Modifizieren der rekonstruierten Oberfläche basierend auf einem berechneten Residuum.

7. Hornhauttopographiegerät, umfassend:
- eine Stimulatorquelle (300), die eingerichtet ist, um in Benutzung eine Zieloberfläche (320), die eine Hornhautoberfläche umfasst, mit einem Lichtmuster zu beleuchten,
- eine Bildzielvorrichtung (310), die eingerichtet ist, um das reflektierte Bild von der Zieloberfläche zu empfangen, wobei das Hornhauttopographiegerät weiterhin eine Rechnereinheit umfasst, die eingerichtet ist für:
- Konstruieren einer rekonstruierten Oberfläche (380), die die Zieloberfläche repräsentiert, durch Prozessieren von Information der Stimulatorquelle und des Bildes, das auf der Bildzielvorrichtung empfangen wird,
- Identifzieren eines Referenzbildpunktes auf der Bildzielvorrichtung, der einem Referenzstimulatorpunkt auf der Stimulationsquelle entspricht,
- Berechnen eines Residuums für den Referenzbildpunkt unter Verwendung der rekonstruierten Oberfläche, welches die Übereinstimmung zwischen der Zieloberfläche und der rekonstruierten Oberfläche repräsentiert,
**dadurch gekennzeichnet, dass** die Rechnereinheit eingerichtet ist für:
- Darstellen des Residuums zusammen mit dem reflektierten Bild auf der Zieloberfläche, die eine Hornhautoberfläche umfasst.

8. Hornhauttopographiegerät gemäß Anspruch 7, wobei die Rechnereinheit eingerichtet ist, um das Residuum zu berechnen durch:
- Selektieren einer Region auf der Bildzielvorrichtung, die den Referenzbildpunkt umgibt,
- Berechnen des zugehörigen Stimulatorursprungspunkts für eine Vielzahl von Bildpunkten innerhalb der Region unter Verwendung von Zurückverfolgen von dem Bildpunkt hin zu der rekonstruierten Oberfläche und von der rekonstruierten Oberfläche hin zu der Stimulatorquelle,
- Selektieren des Bildpunktes dessen zugehöriger Stimulatorursprungspunkt am nächsten zu dem Referenzstimulatorpunkt ist, als der rekonstruierte Referenzbildpunktursprung,
- Bestimmen des Residuums aus der Differenz zwischen dem Referenzbildpunkt, dem Referenzbildpunkt und dem rekonstruierten Referenzbildpunktursprung.

9. Hornhauttopographiegerät gemäß Anspruch 7 oder 8, wobei die Stimulatorquelle eingerichtet ist, um die Zieloberfläche mit einem Schachbrettmuster zu beleuchten.

10. Hornhauttopographiegerät gemäß Anspruch 7 oder 8, wobei die Stimulatorquelle eingerichtet ist, um die Zieloberfläche mit einem farbkodierten Muster zu beleuchten.

11. Hornhauttopographiegerät gemäß einem der Ansprüche 7 bis 10, wobei die Rechnereinheit weiterhin eingerichtet ist, um den Schritt durchzuführen: Modifizieren der rekonstruierten Oberfläche basierend auf dem berechneten Residuum.

12. Hornhauttopographiegerät gemäß Anspruch 11, wobei der Modifikationsschritt eine Anpassung vom Typ kleinstes Quadrat basierend auf dem berechneten Residuum beinhaltet.

13. Verfahren gemäß einem der Ansprüche 1 bis 6, wobei dem Verfahren ein Kalibrationsverfahren vorangeht, das die Schritte umfasst:
Identifizieren eines Referenzbildpunktes auf einem Bild, das durch Reflektieren eines Stimulatorquellmusters auf einer Referenzoberfläche hin zu einer Bildzielvorrichtung erzeugt wird, der einem Referenzstimulatorpunkt entspricht,
Berechnen der Koordinaten des Referenzstimulatorpunkts des Referenzbildpunktes durch rückwärtsgerichtetes Raytracing von dem Bildpunkt hin zu der Referenzoberfläche und von der Referenzoberfläche hin zu der Stimulatorquelle,
Bestimmen der geometrischen Beziehung zwischen dem Referenzbildpunkt und dem Referenzstimulatorpunkt.

14. Hornhauttopographiegerät gemäß einem der Ansprüche 7 bis 12, wobei die Rechnereinheit weiterhin eingerichtet ist, um ein Kalibrationsverfahren in Benutzung durchzuführen, welches die Schritte umfasst:
Identifizieren eines Referenzbildpunktes auf einem Bild, das durch Reflektieren eines Stimulatorquellmusters auf einer Referenzoberfläche hin zu einer Bildzielvorrichtung erzeugt wird, der einem Referenzstimulatorpunkt entspricht,
Berechnen der Koordinaten des Referenzstimulatorpunkts des Referenzbildpunktes durch rückwärtsgerichtetes Raytracing von dem Bildpunkt hin zu der Referenzoberfläche und von der Referenzoberfläche hin zu der Stimulatorquelle,
Bestimmen der geometrischen Beziehung zwischen dem Referenzbildpunkt und dem Referenzstimulatorpunkt.

## Revendications

1. Procédé d'évaluation d'une correspondance entre une surface cible comprenant une surface cornéenne et une surface reconstruite représentant la surface cible, la surface reconstruite étant construite en traitant des informations obtenues en éclairant la surface cible avec un motif de lumière d'une source de stimulateur et en capturant une image réfléchie du motif de lumière sur une cible d'image, le procédé comprenant les étampes :
- de détermination d'un point d'image de référence sur la cible d'image correspondant à un point de stimulateur de référence sur la source de stimulateur,
- de calcul, pour le point d'image de référence, en utilisant la surface reconstruite, d'un reste représentant la correspondance entre la surface cible et la surface reconstruite,
- d'affichage du reste avec l'image réfléchie de la surface cible comprenant la surface cornéenne,
- d'évaluation de la correspondance entre la surface cible et la surface reconstruite à partir du reste affiché.

2. Procédé selon la revendication 1 dans lequel le reste comprend un reste angulaire calculé par suivi arrière du point d'image de référence vers la surface reconstruite et de la surface reconstruite vers la source de stimulateur.

3. Procédé selon la revendication 1, dans lequel l'étape de calcul du reste comprend les étapes :
- de calcul d'une pluralité de points de stimulateur correspondant à une pluralité de points d'image à proximité du point d'image de référence par suivi arrière des points d'image vers la surface reconstruite et de la surface reconstruite vers la source de stimulateur,
- de sélection du point d'image dont le point de stimulateur correspondant est le plus proche du point de stimulateur de référence en tant qu'origine de point d'image de référence reconstruit,
- d'établissement d'un reste à partir de la différence entre le point d'image de référence et l'origine de point d'image de référence reconstruit.

4. Procédé selon la revendication 2 ou 3, dans lequel la construction de la surface reconstruite comprend un ajustement de surface en utilisant des polynômes de Zernike.

5. Procédé selon la revendication 2 ou 3, dans lequel la construction de la surface reconstruite comprend un ajustement de surface en utilisant des fonctions spline.

6. Procédé selon l'une quelconque des revendications précédentes, comprenant en outre l'étape de modification de la surface reconstruite sur la base du reste calculé.

7. Topographe cornéen comprenant :
- une source de stimulateur (300) agencée pour éclairer, en utilisation, une surface cible (320) comprenant une surface cornéenne avec un motif de lumière,
- une cible d'image (310) agencée pour recevoir l'image réfléchie de la surface cible, le topographe cornéen comprenant en outre une unité de calcul agencée pour :
- construire une surface reconstruite (380) représentant la surface cible en traitant des informations de la source de stimulateur et l'image reçue sur la cible d'image,
- identifier un point d'image de référence sur la cible d'image correspondant à un point de stimulateur de référence sur la source de stimulateur,
- calculer, pour le point d'image de référence, en utilisant la surface reconstruite, un reste représentant la correspondance entre la surface cible et la surface reconstruite,
**caractérisé en ce que** l'unité de calcul est agencée pour :
- afficher le reste avec l'image réfléchie de la surface cible comprenant une surface cornéenne.

8. Topographe cornéen selon la revendication 7, dans lequel l'unité de calcul est agencée pour calculer le reste en
- sélectionnant une région sur la cible d'image entourant le point d'image de référence,
- calculant le point d'origine de stimulateur correspondant pour une pluralité de points d'image à l'intérieur de la région en utilisant un suivi arrière du point d'image vers la surface reconstruite et de la surface reconstruite vers la source de stimulateur,
- sélectionnant le point d'image dont le point d'origine de stimulateur correspondant est le plus proche du point de stimulateur de référence en tant qu'origine de point d'image de référence reconstruit,
- établissant le reste à partir de la différence entre le point d'image de référence et l'origine de point d'image de référence reconstruit.

9. Topographe cornéen selon la revendication 7 ou 8, dans lequel la source de stimulateur est agencée pour éclairer la surface cible avec un motif en damier.

10. Topographe cornéen selon la revendication 7 ou 8, dans lequel la source de stimulateur est agencée pour éclairer la surface cible avec un motif codé par couleur.

11. Topographe cornéen selon l'une quelconque des revendications 7 à 10, dans lequel l'unité de calcul est en outre agencée pour exécuter l'étape de modification de la surface reconstruite sur la base du reste calculé.

12. Topographe cornéen selon la revendication 11, dans lequel l'étape de modification comprend un ajustement des moindres carrés basé sur le reste calculé.

13. Procédé selon l'une quelconque des revendications 1 à 6, dans lequel le procédé est précédé d'un procédé d'étalonnage comprenant les étampes :
d'identification d'un point d'image de référence sur une image générée en réfléchissant un motif de source de stimulateur sur une surface de référence vers une cible d'image correspondant à un point de stimulateur de référence,
de calcul des coordonnées du point de stimulateur de référence du point d'image de référence par un suivi de rayon arrière du point d'image vers la surface de référence et de la surface de référence vers la source de stimulateur,
d'établissement de la relation géométrique entre le point d'image de référence et le point de stimulateur de référence.

14. Topographe cornéen selon l'une quelconque des revendications 7 à 12, dans lequel l'unité de calcul est en outre agencée pour effectuer, en utilisation, un procédé d'étalonnage comprenant les étampes :
d'identification d'un point d'image de référence sur une image générée en réfléchissant un motif de source de stimulateur sur une surface de référence vers une cible d'image correspondant à un point de stimulateur de référence,
de calcul des coordonnées du point de stimulateur de référence du point d'image de référence par un suivi de rayon arrière du point d'image vers la surface de référence et de la surface de référence vers la source de stimulateur,
d'établissement de la relation géométrique entre le point d'image de référence et le point de stimulateur de référence.
